Europäisches Patentamt

European Patent Office

Office européen des brevets

(19)

(11) Veröffentlichungsnummer: **0 029 244**
**B1**

(12) **EUROPÄISCHE PATENTSCHRIFT**

(45) Veröffentlichungstag der Patentschrift: **16.04.86**

(21) Anmeldenummer: **80107126.7**

(22) Anmeldetag: **17.11.80**

(51) Int. Cl.⁴: **G 01 T 1/164**

(54) **Verfahren und Gerät zur Korrektur von Ungleichförmigkeiten in den Bildereignis-Energiesignalen einer Szintillationskamera.**

(30) Priorität: **20.11.79 US 96181**

(43) Veröffentlichungstag der Anmeldung:
**27.05.81 Patentblatt 81/21**

(45) Bekanntmachung des Hinweises auf die Patenterteilung:
**16.04.86 Patentblatt 86/16**

(84) Benannte Vertragsstaaten:
**DE FR GB NL**

(56) Entgegenhaltungen:
EP-A-0 002 540
EP-A-0 007 698
EP-A-0 021 366
US-A-3 011 057
US-A-3 745 345
US-A-3 984 689
US-A-4 095 108
US-A-4 151 416

**Die Akte enthält technische Angaben, die nach dem Eingang der Anmeldung eingereicht wurden und die nicht in dieser Patentschrift enthalten sind.**

(73) Patentinhaber: **Siemens Aktiengesellschaft Berlin und München Wittelsbacherplatz 2 D-8000 München 2 (DE)**

(72) Erfinder: **Arseneau, Roger E.**
**511 Ivy Lane**
**Arlington Heights Illinois 60004 (US)**

(56) Entgegenhaltungen:

**Raytheon Medical Electronics, ST-3405, Nov. 1977**

**Journal of Nuclear Medicine 12, 1971, S. 785 bis 791**

**"Removal of Gamma Camera Nonlinearity and Nonuniformities through Real-Time Signal Processing": Druckschrift von der Nuklearmedizin-Konferenz in Paris, July 1979.**

Courier Press, Leamington Spa, England.

## Beschreibung

Die vorliegende Erfindung bezieht sich auf ein Verfahren zur Korrektur von Bildereignis-Energiesignalen einer Szintillationskamera in bezug auf Ungleichförmigkeiten in der Energieverteilungs-Charakteristik der Szintillationskamera, welche Ungleichförmigkeiten eine Funktion der Bildfeldposition der Bildereignisse sind, sowie zur Überprüfung der korrigierten Bildereignis-Energiesignale, wobei die Szintillationskamera neben dem Bildereignis-Energiesignal auch ein Bildereignis-Positionskoordinatensignal in Antwort auf jedes auftretende Bildereignis erzeugt, wobei wenigstens ein vorgegebene Grenzen aufweisendes Energiefenster erzeugt wird, das außerhalb der vorgegebenen Grenzen liegende Bildereignis-Energiesignale verwirft, wobei eine Matrix von Korrekturfaktoren während einer off-line Test-, Meß- und Analysierphase bestimmt wird, indem die energieabhängige Antwort der Szintillationskamera auf eine eine gleichmäßige Strahlungsverteilung aufweisende Teststrahlungsquelle gemessen wird, wobei die Korrekturfaktoren jeweils eine Änderung der energieabhängigen Antwort der Szintillationskamera als Funktion der Bildfeldposition repräsentieren, wobei die so bestimmten Korrekturfaktoren zum Gebrauch während einer on-line Untersuchungsphase der Szintillationskamera gespeichert werden, wobei während der on-line Untersuchungsphase jeweils derjenige Korrekturfaktor, der zur gerade gebildeten Bildereignis-Positionskoordinatensignal gehört, ausgelesen wird.

Die Erfindung bezieht sich weiterhin auf eine Vorrichtung zur Durchführung dieses Verfahrens, mit einer Szintillationskamera, die Bildereignis-Positions-koordinatensignale und Bildereignis-Energiesignale für diejenigen Bildereignisse liefert, die während der on-line Untersuchungsphase der Szintillationskamera auftreten, mit einem Speicher, in dem in der off-line Test-, Meß- und Analysierphase ermittelte Korrekturfaktoren in einer adressierbaren Daten-Matrix gespeichert sind, wobei diese Korrekturfaktoren jeweils eine Änderung der energieabhängigen Antwort des Szintillationskamera als Funktions der Bildfeldposition repräsentieren, mit einer Steuereinheit, die auf das Bildereignis-Positionskoordinatensignal eines jeden Bildereignisses hin den Speicher in der Weise steuert, daß er den Korrekturfaktor entsprechend dem gerade anliegenden Bildereignis-Positionskoordinatensignal ausliest, mit einer Korrektureinheit für das Bildereignis-Energiesignal, die in Abhängigkeit vom Bildereignis-Energiesignal und dem ausgelesenen Korrekturfaktor ein korrigiertes Bildereignis-Energiesignal liefert, und mit einem Energie-Analysator, der in Energiefenster mit vorgegebenen Grenzen aufweist, dem das korrigierte Bildereignis-Energiesignal zugeführt ist und der die außerhalb der vorgegebenen Grenzen liegende Bildereignis-Energiesignale verwirft.

Verzerrungen in enem Bild aus Bilddaten, die von einer Szintillationskamera und einem beigeordneten Bildaufzeichnungsgerät erhalten werden, sind primär räumliche Verzerrungen und Änderungen in der Punktquellen-Empfindlichkeit (Ungleichförmigkeiten im Bildereignis-Energiesignal). In den derzeit verfügbaren Szintillations-kameras vom "Anger-Typ" werden Änderungen der Punktquellen-Empfindlichkeit durch besonderes Bauteilgestaltung und durch besonders sorgfältige Abstimm- und Einjustiermaßnahmen innerhalb 1 bis 2% gehalten. Darüber hinaus sind auch schon Korrekturmethoden bekannt, die eine Korrektur der räumlichen Verzerrung erlauben; hier werden also Bildereignisse korrigiert, die von der Szintillationskamera nicht in ihrer korrekten Bildereignislage in bezug auf das Gesamtbild und die Position des orginalen Auftretens erfaßt werden. Ein solches Verfahren zur Korrektur der rämlichen Verzerrung ist z. B. in US—A— 3,745,345 beschrieben.

Ein anderes Korrekturverfahren dieser Art, das noch genauer arbeitet, sowie das zugehörige Korrekturgerät sind Gegenstand der am 20. Juni 1980 eingereichten europäischen Patentanmeldung Nr. 80 103 460.4 (Priorität 22.05.1979; Veröffentlichungstag: 07.01.1981; Veröffentlichungsnummer: 0,021,366). Dieses Verfahren sieht eine genaue Rückversetzung jedes Ereignisses vor, und es korrigiert die räumliche Verzerrung so, daß ein korrigiertes Bild erhalten wird, das Änderungen der Flächendichte von höchstens etwa 1% als Antwort auf eine gleichförmige Flutungsquelle beinhaltet.

Das Aufkommen besserer Korrekturverfahren für die räumliche Verzerrung in Bildern von Szintillationskameras hat zum Ergebnis eine gewachsene Aufmersamkeit betreffend Probleme und Verbesserungen bei Szintillationskameras auf dem Gebiet von Nichtlinearitäten, die durch Änderungen in der Energieverteilung der Bildereignissignale als Funktion der Position auf der Bildfläche der Kamera verursacht werden; diese Änderungen sind auch als Änderungen der Punktquellen-Empfindlichkeit bzw. als Ungleichförmigkeiten des Bildereignis-Energiesignals bekannt.

Es ist zwar möglich, durch rein bauliche Maßnahmen die Ungleichförmigkeiten der Energieverteilung bei Szintillationskameras auf 1 bis 2% zu begrenzen; dadurch steigen aber die Herstellungskosten, gerade wegen der besonderen Bauteilgestaltung und der sorgfältigen Abstimmaßnahmen, die durchgeführt werden müssen. Darüber hinaus sind die Anforderungen an die einzelnen Bauteile von Szintillationskamera, insbesondere an den Szintillationskristall des Kameradetektorkopfes, ziemlich scharf; das Ergebnis ist, daß solche Bauteile teuer werden und daß die Ausschußrate bei der Fertigung größer ist als gewünscht.

Darüber hinaus ist aber auch noch folgendes zu bedenken:

Durch eine geeignete Ausbildung und durch eine besondere Abstimmung lassen sich

Ungleichförmigkeiten in der Energieverteilungs-Charakteristik auf etwa 1 bis 2% reduzieren, aber nur dann, wenn sich die ungleichförmige Charakteristik in konstantem Maßstab ändert. Für Ungleichförmigkeiten des Kristalls, die auf abrupten Änderungen, verursacht z.B. durch Sprung- oder Stoßstellen, innerhalb kleiner Flächenbereiche des Kristalls beruhen, besteht wärend des Herstellungsprozesses keine Möglichkeit des Ausgleichs oder der Kompensation.

Wird hingegen zusätzlich oder allein eine arbeitsintensive Feinabstimmung durchgeführt, um auf diese Weise eine gleichmäßige Verteilung der Bildereignis-Energiesignale quer über die Bildfläche der Kamera zu erhalten, so wird die Kamera anfällig für Verstimmeffekte während ihrer Lebensdauer, so daß häufige Nachstimmungen nötig sind, die unangenehme Nachfolgeeffekte haben können, aus denen unter Umständen ungenaue Bildanzeigen resultieren.

Ein anderes Problem betreffend Ausgestaltung, Herstellung und Abstimmung der Kamera, um eine möglichst gleichförmige Charakteristik zu erhalten, ist, daß ein Kompromiß gefunden werden muß zwischen einzelnen Eigenschaften der Kamera, wie z.B. Ungleichförmigkeiten gegenüber Punktquellen Empfindlichkeit, räumlicher Verzerrung und Auflösung. Ändert man nun jedoch einzelne Eigenschaften, um die gewünschte Gleichförmigkeit in der Energieverteilung quer über das Bildfeld der Kamera zu erhalten, so bleiben andere wichtige Kameramerkmale davon nicht unbeeinflußt. Sie ändern sich ebenfalls, und zwar in unter Umständen ungünstigem Maße.

Szintillationskameras, deren räumliche Verzerrungen so weit korrigiert sind, daß die Schwankungen der Bildflächendichte nur noch bei etwa 1% liegen, sind für den diagnostischen Gebrauch nur dann von Wert, wenn die Ungleich-förmigkeiten, die aufgrund von Energie-änderungen bewirkt werden, genauso akkurat korrigiert sind; Bildereignisse, die übermäßige Änderungen des Bildereignis-Energiesignals hervorrufen, werden mit Sicherheit ausrangiert, wenn das Bildereignis-Energiesignal sich außerhalb des Energiefensters der Szintillationskamera befindet, auch wenn diese Bildereignisse an sich als gültig zu bewerten sind. Die Verwendung eines breiteren Energiefensters zur Annahme von Bildereignissen kann jedoch dazu führen, daß Bildereignisse angenommen werden, die durch Streustrahlung verursacht werden. Dadurch wird also immer noch nicht eine gleichförmige Annahme von Bildereignissen quer über die Stirnfläche der Kamera erreicht.

Verschiedene Verfahren zur Korrektur der Ungleichförmigkeiten von Szintillationskameras sind bereits vorgeschlagen worden.

Diese Verfahren beruhen alle auf unterschiedlichen Versuchen, das Energiefenster, mit dem jedes Bildereignissignal verglichen wird, zu variieren, um so bestimmen zu können, ob das Bildereignissignal als gültiges Bildereignis weiterverarbeitet und gemäß den angegebenen X, Y-Bildkoordinaten angezeigt werden soll. Sie rangieren alle unter dem Begriff des sogennannten "gleitenden Energiefensters".

Ein solches Verfahren zur Variation des Energiefensters ist z.B. durch G. F. Knoll et al in der Schrift "Removal Of Gamma Camera Nonlinearity And Nonuniformities Through Real-Time Signal Processing" beschrieben, die im Juli 1979 auf der Nuklearmedizin-Konferenz in Paris, Frankreich, präsentiert wurde.

Gemäß dieser Schrift sind die oberen und unteren Grenzwerte eines Z(Energie)-Fensters in digitaler Form entsprechend der Aufteilung der Kamerabildfläche in einer 64×64 Datenmatrix gespeichert. Wenn also von der Kamera die X, Y-Position eines Bildereignisses ausgegeben wird, so werden gleichzeitig auch die zu dieser Position gehörenden unteren und oberen Grenzwerte des Energiefensters zu einer Fenstervergleichsstufe ausgelesen, in der das Z-Bilder-eignissignal mit dem programmierten Energie-fenster verglichen wird.

Andere Studien, die sich mit der Korrektur der Ungleichförmigkeit befassen, sind "Sources of Gamma Camera Image Inequalities" von Morrison et al, Journal of Nuclear Medicine 12, Seiten 785—791, 1971, and "A New Method Of Correcting For Detector Non-Uniformity In Gamma Camera" von Lapidus, Raytheon Medical Electronics, ST-3405, November 1977.

Der Lapidus-Artikel beschreibt dabei ein Verfahren zum Modifizieren des Z(Energie)-Signals eines Bildereignisses dadurch, daß ein-gespeicherter Korrekturfaktor (von einem 64×64 Datenfeld) entsprechend der Position des Bilder-eignisses ausgelesen wird. Der Korrekturfaktor verändert die Pulsbreite des Z-Signals unter Zuhilfenahme eines Pulsbreitenmodulators, um ein Z-Signal mit variabler Pulsbreite zu erhalten. Das Bildgerät benutzt dieses Z-signal mit variabler Pulsbreite dazu, die Intensität des abgebildeten Bildpunktes auf einem Film zu variieren. Ein Flutung-Vorgang wird durchgeführt, um die Verteilung der Ungleichförmigkeiten zu "erlernen". Es wird so ein Datenfeld von Werten erhalten und gespeichert, das die Antwort des Kameradetektors in jeder der insgesamt 4096 X, Y-Positionen auf die Flutung repräsentiert.

Die soeben angeführten Verfahren und Vorschläge gestatten keine Energiekorrektur in dem Sinne, daß ein Bildereignis-Energiesignal mit den Grenzen eines festen Energiefensters verglichen wird. Der abgehandelte Stand der Technik stellt auch kein System zur Verfügung, das fähig ist automatisch mit einer Mehrzahl unterschiedlicher Strahlungsquellen zu arbeiten und dabei die Ungleichförmigkeiten der Kamera zu korrigieren. Auch sind noch keine Systeme bekannt, die mit Strahlungsquellen zusammenarbeiten, von denen jede einzelne eine Vielzahl von Energieniveaus beinhaltet.

Ein Verfahren und eine Vorrichtung der eingangs genannten Art sind aus der US—A—4,095,108, insbesondere Figur 6, bekannt. Dort ist in der Beschreibung pauschal angegeben, daß zur Korrektur der Energievertilungs-Charakteristik das Bildereignis-Energiesignal jeweils mit einem Wert oder Faktor belegt wird, der entweder hinzuaddiert oder mit dem multipliziert wird. In der Figur 6 ist lediglich die Ausführungsform des Addierens dargestellt. In beiden Fällen, d.h. sowohl beim Addieren als auch beim Multiplizieren, wird auch hier ein gleitendes Energiefenster erzeugt. Als Nachteil eines solchen variablen Energiefensters wird der damit verbundene Aufwand und die verhältnismäßig geringe Verarbeitungsgeschwindigkeit für die Bildereignis-Energiesignale angesehen.

Aufgabe der vorliegenden Erfindung ist es, ein Verfahren und ein Vorrichtung der eingangs genannten Art dahingehend weiterzubilden, daß ein festes Energiefenster benutzt werden kann. Insbesondere soll es auch ohne Bedeutung sein, mit wievielen einzelnen Strahlungsquellen die Szintillationskamera arbeitet oder wieviele strahlende Energieniveaus eine einheitliche Strahlungsquelle besitzt.

Die Aufgabe wird bei einem Verfahren der eingangs genannten Art erfindungsgemäß dadurch gelöst, daß jeweils das Bildereignis-Energiesignal für das betreffende Bildereignis dadurch korrigiert wird, daß das Bildereignis-Energiesignal mit dem gerade ausgelesenen Korrekturfaktor multipliziert und daß zu dem Produkt das Bildereignis-Energiesignal addiert wird, wodurch ein korrigiertes Bildereignis-Energiesignal erhalten wird, und

daß dieses korrigierte Bildereignis-Energiesignal dem Energiefenster zugeleitet wird, welches fest eingestellte Grenzen aufweist.

Eine Vorrichtung der eingangs genannten Art zur Durchführung dieses Verfahrens zeichnet sich erfindungsgemäß dadurch aus, daß die Korrektureinheit Multipliziermittel umfaßt, um den ausgelesenen Korrekturfaktor mit dem Bildereignis-Energiesignal zu multiplizieren, sowie Mittel zur Addition, um das Bildereignis-Energiesignal und das Produkt aus dem Bildereignis-Energiesignal und dem ausgelesenen Korrekturfaktor zum korrigierten Bildereignis-Energiesignal zusammenzufassen, und daß der Energie-Analysator ein Energiefenster mit fest eingestellten Grenzen aufweist.

Die Erfindung erlaubt es also, daß Ungleichförmigkeiten in der Energieverteilungs-Charakteristik bei der Szintillationskamera direkt am Bildereignis-Energiesignal korrigiert werden. Die Korrekturfaktoren werden dabei in der off-line Test-, Meß- und Analysierphase, die der on-line Untersuchungsphase vorausgeht, über das gesamte Bildfeld der Kamera ermittelt. Hierzu wird die Bildfläche in eine Matrix aus einzelnen Bildelementflächen unterteilt; für jedes Bildelement wird der Korrekturfaktor ermittelt. Das sich somit ergebende Datenfeld wird dann als Funktion der Position der Bildereignisse und adressierbar durch Bildereignisse gespeichert. Während des on-line Betriebes der Szintillationskamera und des beigeordneten Bildanzeigesystems wird der passende, abgespeicherte Energiekorrekturfaktor, der einem Bildereignis entspricht, zur on-line Energiekorrektureinheit ausgelesen. Die on-line Korrektureinheit benutzt das Bildereignis-Energiesignal und den zugehörigen gespeicherten Korrekturfaktor dazu, das Bildereignis-Energiesignal direkt zu korrigieren. Das korrigierte Bildereignis-Energiesignal wird dann dem Energie-Analysator zugeführt, der ein Energiefenster aufweist, das eine fest eingestellte Breite hat. Der Analysator bestimmt durch Vergliech, ob das korrigierte Signal in den Akzeptierbereich des Fensters paßt, so daß das Bildereignis für die Abbildung zugelassen werden kann, oder ob es nicht in das Fenster paßt, so daß das Bildereignis abgelehnt werden muß.

In der off-line Test-, Meß- und Analysierphase werden die Korrekturfaktoren mittels einer Strahlungsquelle ermittelt, die ein einziges Energieniveau aufweist. Die so ermittelten Korrekturfaktoren können dann in nachfolgenden on-line Betrieb zur Korrektur eingesetzt werden, wobei es unerheblich ist, ob das zu korrigierende Bildereignis-Energiesignal von einer einzelnen Strahlungsquelle stammt, die mehrere unterschiedliche Energieniveaus aufweist, oder ob es von einer Vielzahl von Einzelquellen herrührt, die unterschiedliche Energieniveaus haben.

Weiter Vorteil und Einzelheiten der Erfindung ergeben sich aus der nachfolgenden Beschreibung eines Ausführungsbeispiels anhand der Zeichnung und in Verbindung mit den Abhängigen Ansprüchen.

Es zeigen:

Figur 1 die Erfindung als Bestandteil in einem Prinzipschaltbild,

Figuren 2, 3 und 4 Diagramme von möglichen Energieverteilungen, und

Figur 5 dei Erfindung in detaillierter Darstellung als Ausschnitt aus dem Prinzipschaltbild der Figur 1.

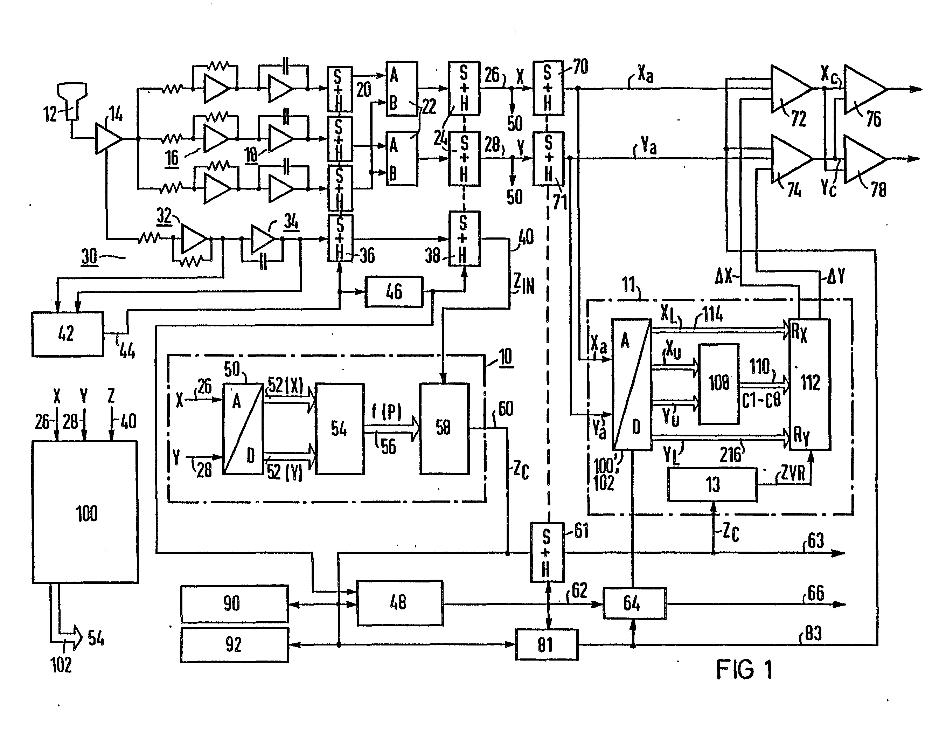

Ein on-line Energiekorrigiergerät, das dazu benutzt wird, das vorliegende erfindungsgemäße Verfahren zu praktizieren, hat in der Figur 1 die Bezugsziffer 10. Es ist dargestellt in Verbindung mit Teilen einer Szintillationskamera. Die Szintillationskamera der Figur 1 ist von dem Typ, der Szintillationsereignisse in elektrische Signale umwandelt, die die Ortskoordinaten jedes des Szintillationsereignisse und deren Energie repräsentieren. Dieser Type ist z.B. auch als "Anger-Typ" gut bekannt. Solche Szintillationskameras sind beispielsweise in den US-Patenten US—A—3,011,057, US—A—3,745,345 und US—A—3,984,689 beschreiben, auf die im Hinblick auf weitere Information hinsichtlich Aufbau und Funktionsweise einer Szintillationskamera ausdrücklich Bezug genommen wird.

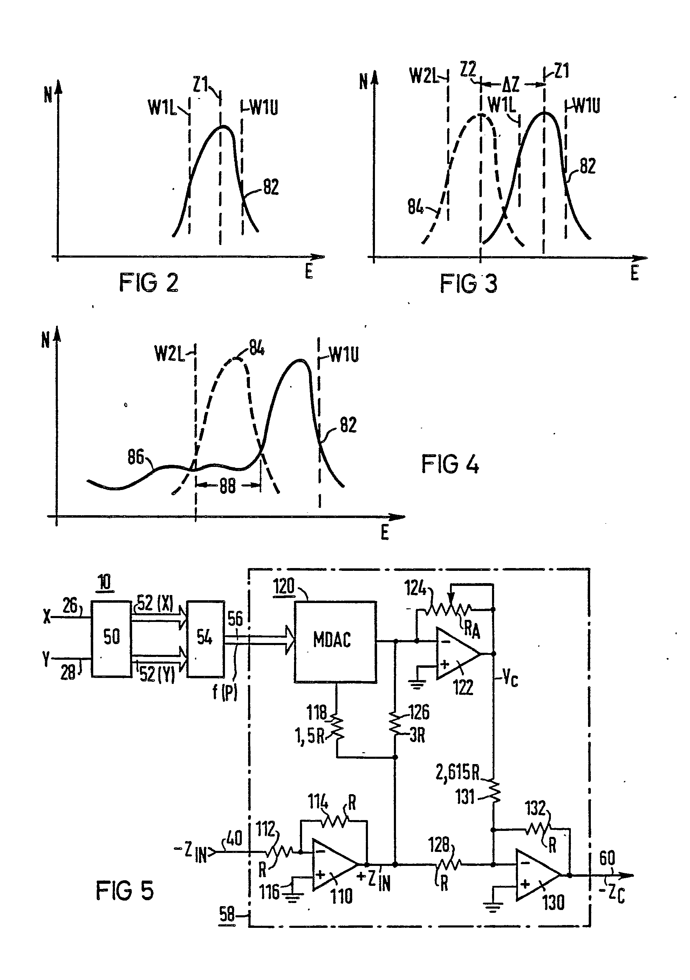

Szintillationsereignisse treten grundsätzlich immer dann auf, wenn z.B. Gammastrahlen auf einen Szintillatonskristall an der Stirnfläche der Kamera auftreffen. Sie lassen sich mittels Fotovervielfacherröhren (eine davon ist in der Figur 1 z.B. mit der Kennziffer 12 angedeutet) erfassen, wozu diese in einem vorbestimmten Konfigurationsmuster hinter dem Szintillationskristall angeordnet sind, um so die Lichtenergie jedes Szintillationsereignisses in elektrische Pulse umformen zu können. Es gibt besondere Ausgestaltungen von Szintillationskameras, bei denen zwischen dem Kristall und den Fotovervielfacherröhren auch noch eine Anordnung von Lichtröhren vorhanden ist. Normale Szintillationskameras arbeiten mit Gruppen von etwa 37 bis 75 Fotovervielfacherröhren, um so die Szintillationsereignisse quer über die Oberfläche des Szintillationskristalles erfassen zu können, so wie es beispielsweise in den Figuren 1 und 2 des vorher erwähnten US-Patentes US—A—3,745,345 dargestellt ist.

In Übereinstimmung mit üblicher Detektorelektronik von Szintillationskameras sind die Pulsausgänge aller Fotovervielfacherröhren 12 innerhalb der Röhrenanordnung über Vorverstärker 14, Summier- und Subtrahierverstärker 16, Integrationsstufen 18, Abtast- und Haltestufen 20 und Multiplzierstufen 22 mit Halte- und Abtaststufen 24 verbunden, die, so wie es in der Figur 1 grundsätzlich dargestellt ist, an ihren Ausgängen 26, 28 die X- und Y-Positionskoordinaten eines jeden Szintillationsereignisses liefern. Das Ausgangssignal einer jeden Fotovervielfacherröhre in der Röhrenanordnung hängt ab von der Nähe der betroffenen Fotovervielfacherröhre zum Szintilationsereignis.

Die Ausgangssignale der Fotovervielfacherröhren 12 werden auch noch einem Energieanalysierkreis 30 für das Z-Signal zugeleitet. Der Kreis 30 umfaßt dabei eine Summierverstärkerstufe 32, eine Integrationsstufe 34 und zwei Abtast- und Haltestufen 36 und 38. Der Ausgang der Abtast- und Haltestufe 38 erzeugt bei 40 ein unkorrigiertes Z-Signal $Z_{IN}$, das die Energie eines jeden Bildereignisses, so wie es anfällt, repräsentiert. Der Kreis 30 für das Z-Signal umfaßt aber auch noch einen Verarbeitungskreis 42, der an den Ausgängen des Summierverstärkers 32 und des Integrators 34 liegt und der bei 44 ein Ausgangssignal für die Abtast- und Haltestufe 36 sowie für eine Entzerr- und Steuerstufe 46 erzeugt. Der Verarbeitungskreis 42 versorgt das Gerät mit der Funktion eine eingangsseitigen Grobanalysators, so wie er z.B. in der US—A—3,984,689 nach Aufbau und Funktionsweise beschrieben ist. Der Ausgang der Entzerr- und Steuerstufe 46 geht einerseits auf die Abtast- und Haltstufe 38 und andererseits auf einen Energie-Analysator 48, der ein Energiefenster mit bestimmter Breite aufweist. Die X- und Y-Koordinatensignale bei 26 und 28 und das Z-Energiesignal $Z_{IN}$ bei 40 liegen als Eingangssignale am Energiekorrekturgerät 10.

Das on-line Energiekorrekturgerät 10 umfaßt einen Analog-Digital-Konverter 50, der mit den Ausgängen 26 und 28 für die X- und Y-Positionskoordinatensignale verbunden ist. Der Analog-Digital-Konverter 50 liefert an den Ausgängen 52 die X- und Y-Positionskoordinate für jedes Bildereignis als Digitalwert auf einer geeigneten Anzahl von digitalen Steuerleitungen. Die digitalen Ausgangswerte werden auf den Addressiereingang eines Energiekorrekturfaktor-Speichers 54 gegeben. Der Speicher 54 hat ein Datenfeld von Energiekorrigierfaktoren gespeichert, die innerhalb des Datenfeldes so angeordnet sind, daß sie in Einklang mit Informationen über die Koordinaten von Bildfeldpositionen adressierbar sind, welche Informationen Flächenteile oder Bildträger des Bildfeldes der Kamera repräsentieren. Die Energiekorrekturfaktoren werden in einer off-line Test-, Meß- und Analysierphase bestimmt und im Energiekorrekturfaktor-Speicher 54 gespeichert zum Zwecke der Benutzung während der on-line Untersuchungsphase der Szintillationskamera und des zugeordneten Apparates.

Während der on-line Untersuchungsphase der Szintillationskamera ruft jedes aus einer Position X, Y anfallende Bildereignis über die zugehörigen digitalisierten X- und Y-Koordinatensignale im Energiekorrekturfaktor-Speicher 54 eine Adresse für eine Speicherzelle an, in der ein Energiekorrekturfaktor gespeichert ist, der speziell dem Bildereignis aus der Position X, Y zu dessen Energiekorrektur zugeordnet ist. Der Energiekorrekturwert wird daraufhin vom Speicher ausgelesen. Der Energiekorrekturfaktor-Speicher 54 liefert demgemäß also in Antwort auf jedes vorbestimmten Zahl von digitalen Steuerleitungen und in einem digitalen Format, das den Energiekorrekturfaktor so repräsentiert, daß er in geeigneter Weise dem Energiesignal bei 40, das ebenfalls vom Bildereignis stammt, in Übereinstimmung mit dem Bildpunkt, an dem das Ereignis in der Bildfläche aufgetreten ist, zur Durchführung der Korrektur zugeführt werden kann.

Der digitale Korrekturfaktor, der in Figur 1 mit f(P) bezeichnet ist, wird dazu vom Ausgang 56 des Energiekorrekturfaktor-Speichers 54 auf den Eingang einer Energiesignal-Korrektureinheit 58 des Energiekorrekturgerätes 10 gegeben. Gleichzeitig wird auch das Z-Signal $Z_{IN}$ von 40 als Eingangssignal der Korrektureinheit 58 zugeleitet. In Einklang mit dem digitalen Korrekturfaktor f(P) bei 56 und dem analogen Z-Signal $Z_{IN}$ bei 40 liefert daraufhin die Energiesignal-Korrektureinheit 58 an einem Ausgangs 60 ein korrigiertes Energiesignal $Z_C$ in analoger Form. Das korrigierte Energiesignal $Z_C$ bei wird dem Eingang des Energie-Analysators 48 zugeführt.

Der Energie-Analysator 48 vergleicht nun das Energieniveau des korrigierten Energiesignals $Z_C$ bei 60 für jedes Beildereignis mit einem vorgewählten Energiefenster, das durch untere und obere Energiegrenzen definiert ist. Als Ergebnis des Vergleiches erzeugt der Analysator 48 ein Akzeptiersignal am Ausgang 62, wenn das korrigierte Energiesignal $Z_C$ innerhalb der Grenzen des vorgewählten Energiefensters liegt. Dementsprechend liefert er aber am Ausgang 62 ein Ablehnsignal, wenn sich das Energiesignal außerhalb der Grenzen des Energiefensters befindet.

Der Ausgang 62 des Energie-Analysators 48 ist an einer Helltaststeuerstufe 64 angeschlossen, die das beigeordnete Bildanzeige- und Analysegerät über den Helltastausgang 66 steuert. Die Helltaststeuerstufe 64 bestimmte in Antwort auf angenommene oder abgelehnte Signale in der Leitung 62, ob ein Bildereignis angezeigt bzw. gegebenenfalls für analytische Zwecke gezählt werden soll oder nicht.

Die positionskoordinaten des Ereignisses, das angezeigt werden soll, werden von den X; Y-Positionskoordinatentsignalen bei 26, 38 abgeleitet. Durch sie wird schließlich die jeweils richtige Position des Bildereignisses in der Bildmatrix festgelegt, die durch das beigeordnete Bildanzeige- und Analysegerät produziert wird.

Im besonderen ist der Ausgang 26 für das X-Positionskoordinatensignal mit einer Abtast- und Haltestufe 70 verbunden. Diese erzeugt ein Signal $X_a$ für einen ersten Eingang eines X-Misch- und Spektrumtors 72. In ähnlicher Weise ist der Ausgang 28 für das Y-Positionskoordinatensignal mit einer Abtast- und Haltestufe 71 verbunden, die ein Signal $Y_a$ erzeugt, das dem ersten Eingang eines Y-Misch- und Spektrumtores 74 zugeführt wird. Die Ausgänge der Misch- und Spektrumtore 72 und 74 sind mit je einem von zwei Orientierungstoren 76 und 78 verbunden. Die Ausgänge der Tore 76 und 78 steuern direkt die Vertikal- und Horizontalablenkung, über die schließlich die darzustellende Position des Bildereignisses im Bild des Bildgerätes oder am Analysegerät festgelegt wird. Das X-Misch- und Spektrumtor 72 besitzt auch noch einen zweiten Eingang, der mit einem ersten Ausgang $\Delta X$ eines Korrekturgerätes 11 zur Korrektur der räumlichen Verzerrung verbunden ist. In ähnlicher Weise liegt auch das Y-Misch- und Spektrumtor 74 mit einem zweiten Eingang an einem zweiten Ausgang $\Delta Y$ des Korrekturgerätes 11. Ein dritter Eingang für jedes der beiden Tore 72 und 74 ist darüber hinaus mit dem Ausgang 83 einer Steuerstufe 81 für eine Spektrumanzeige verbunden, die einerseits vom Ausgangssignal des Energie-Analysators 48 und andererseits vom korrigierten Energiesignal $Z_c$ kontrolliert wird.

Das Korrekturgerät 11 für die räumliche Verzerrung beinhaltet einen Analog-Digital-Konverter 100', 102' für die Signale $X_a$ und $Y_{a,}$ einen Speicher 108 für Korrekturfaktoren zur Korrektur der räumlichen Verzerrung, einen Korrektur-Interpolator 112 und eine Einheit 13 zur Änderung der Korrektur einer Verzerrung. Es ändert in Übereinstimmung mit dem im Speicher 108 eingespeicherten Korrekturfaktoren für die räumliche Verzerrung und in Abhängigkeit von Adressiersignalen $X_U$, $Y_U$, $X_L$, $Y_L$ die $X_a$, $Y_a$-Positionskoordinaten jedes Bildereignisses, um eine Korrektur der räumlichen Verzerrung in der Bildanzeige zu erhalten.

Während der on-line Untersuchungsphase korrigiert also das Energiekorrekturgerät 10 das unkorrigierte Z-Energiesignal bei 40 in Übereinstimmung mit einem Korrekturfaktor f(P), um so die Ungleichförmigkeit der Energie antwort der Szintillationskamera als eine Funktion der Position des Bildereignisses auf der Kamerabildfläche zu minimieren. Das Energiekorrekturgerät 10 liefert daraufhin eine korrigiertes Energiesignal $Z_c$ bei 60 zum Energie-Analysator 48, der schließlich entscheidet, ob das Bildereignis zur Anzeige angenommen oder abgelehnt wird. Zwecks weiterer Erläuterung wird nun auf die Figur 2 Bezug genommen, in der die Energieantwort einer Szintillationskamera, so wie sie sich für das unkorrigierte Energiesignal darstellt, illustriert ist.

Das Diagram der Figure 2 zeigt die Energie E als Abszisse und die Zahl N der Ereignisse als Ordinate. Die Kurve 82 repräsentiert eine Energieverteilung oder ein Spektrum in einem kleinen ausgewählten Flächenausschnitt 1 auf der Bildfläche der Kamera in Antwort auf eine gleichförmige Flutungsquelle für einen bestimmten Zeitabschnitt, der für eine statische genaue Abtastung ausreichend ist. Die Energieverteilung 82 gruppiert sich um einen Hauptenergiewert Z1, und sie illustriert, daß die meisten Bildereignisse in einem relativ schmalen Bereich ($\pm 6\%$ Halbwertsbreite) um dem Hauptwert Z1 herum in einer Gauss'schen Verteilung liegen. So repräsentiert also die Energieverteilungskurve 82 die Antwort der Szintillationskamera auf eine radioaktive quelle Flutungs in einem Punkt oder in einer vorbestimmten kleinen Fläche, nämlich dem Flächen ausschnitt 1 auf der Bildfläche der Szintillationskamera.

Was nun die Energieverteilungen an anderen Punkten i der Bildfläche der Kamera betrifft, so sind diese in Einklang mit den Ungleichförmigkeiten der Antwort der Szintillationskamera an den unterschiedlichen Flächenelementen von ähnlicher Form; sie haben jedoch unterschiedliche Hauptenergiewerte $Z_i$. Die Änderung in der Antwort hängt von der Art der Herstellung und Abstimmung der Kamera ab.

In der Figure 3 ist neben der Energieverteilungskurve 82 eine zweite Energieverteilungskurve 84 gestrichelt eingezeichnet. Die Energieverteilungskurve 84 repräsentiert beispielsweise die Antwort der Kamera auf einen zweiten Flächenausschnitt 2 der Kamerabildfläche, der zu jenem unterschiedlich ist, zu dem die Energieverteilungskurve 82 gehört. Die beiden Vertilungskurven 82 und 84 illustrieren also die Ungleichförmigkeit der Antwort der Kamera auf Bildereignisse, die von zwei unterschiedlichen Bildelementen oder Flächenausschnitten 1 und 2 der Kamerabildfläche stammen. Der Abstand zwischen den Hauptenergiewerten Z1 und Z2 beider Kurven ist $\Delta Z$ angegeben.

Im on-line Gebrauch der Kamera wird nun der Energiewert des Z-Energiesignals eines jeden Bildereignisses vom Energie-Analysator 48 darauf geprüft, ob er in dem Fensterbereich eines eingestellten Energiefensters fällt. Hierzu muß jedoch das Energiefenster erste einmal auf eine geeignete Breite eingestellt werden.

Wünschenswert ist, daß das Energiefenster auf eine solche Breite eingestellt wird, die einen hohen Prozentsatz aller vorkommenden Ereignisse in der Energieverteilung einschließt;

auf diese Weise ergibt sich ein statistisch genaues Ergebnis, das wieder eine exakte Abbildung der Ereignisse gewährleistet.

In der Figur 2 sind z.B. der untere und obere Wert der Grenzen des Energiefensters mit W1L und W1U bezeichnet. Alle Bildereignisse, die Energiewerte haben, die innerhalb dieser Grenzen W1L und W1U des Fensters liegen, werden akzeptiert und können zur Bildaufzeichnung gelangen. Tritt hingegen ein Bildereignis auf, dessen Energiewert außerhalb dieser Grenzen W1L und W1U liegt so wird das Bildereignis zurückgewiesen; es gelangt nicht zur Aufzeichnung.

Im Falle der Figur 3 würde demnach also die Energieverteilungskurve 84 außerhalb der Grenzen W1L und W1U des auf die Verteilungskurve 82 eingerichteten Energiefensters liegen. Die Ereignisse, die zur Energieverteilungskurve 84 gehören, würden also nicht zur Anzeige gelangen, weil das Energiefenster die Energiewerte für eine Aufzeichnung nicht akzeptieren würde. Auf diese Weise würden also an sich gültige Bildereignisse verworfen werden. Das Bild zeigt sogenannte "kalte Punkte", wo immer die Kamera eine unterschiedliche Energieantwort aufweist. Somit verursachen also die Ungleichförmigkeiten der Kameraantwort Ungleichförmigkeiten im Bildaufbau und zwar als Folge von Energieänderungen quer über die Bildfläche der Kamera. Es ist zu beachten, daß bei üblichen Kameras, bei denen keine besonderen Maßnahmen für eine Kompensation vorgesehen sind, die Änderung der Antwort quer über die gesamte Kamerabildfläche in der Größenordnung 5 bis 10% liegt.

Um dieses Problem zu lösen, könnte beispielsweise daran gedacht werden, die untere Grenze W1L des Energiefensters weiter nach unten zu verschieben, so daß sich eine neue untere Grenze W2L ergibt. Das Energiefenster würde dadurch breiter, und der Energie-Analysator könnte jetzt Energiewerte akzeptieren, die entweder aus der Energieverteilungskurve 82 oder auch aus der Energieverteilungskurve 84 stammen.

Die Verbreiterung des Energiefensters würde aber zu einem anderen Nachteil führen. Wie in der Figur 4 dargestellt ist, besteht jetzt die Gefahr, daß bestimmte Kurvenanteile der Energieverteilungskurve, die allein auf Streustrahlung beruhen, in das Energiefenster fallen, wie es z.B. mit dem Anteil 88 des Steukurvenausläufers 86 der Energieverteilungskurve 82 angedeutet ist. Die Streustrahlung stammt dabei von einem Flächenausschnitt, der an einem anderen Bildfeldort liegt, dessen herabgesetzte Energieniveaus aber noch bis in den Bereich des Flächenausschnittes 1 für die Energieverteilungskurve 82 reichen. Zu solchen Streueffekten kommt es z.B., wenn der Gammastrahl aus seiner ursprünglichen Richtung mit reduzierter Energie abgelenkt wurde, z.B. dadurch, daß er einen Knochen oder einen anderen Teil des Patientenkörpers gestreift hat.

Überlagerte Streueffekte führen also zu fehlerhafter Bildanzeige. Um den Einfluß von Streustrahlung zu vermeiden, muß also vermieden werden, daß solche Streuanteile als gültige Bildereignisse gezählt und im Bild dargestellt werden. Nur so kann vermieden werden, daß es zu unterwünschten Intensitätserhöhungen an einzelnen Bildpunkten im Gesamtbild kommt. Die Anwendung eines breiteren Fensters, so wie es die Figur 4 vorschlägt, ist zur Lösung des Energieantwortproblems also denkbar ungeeignet.

Vorliegende Erfindung löst nun das Problem auf wenig aufwendige Weise. Dazu wird, wir vorher schon erwähnt, eine spezielle Energiekorrektur durchgeführt. Dies geschieht in der Weise, daß Ereignisse, die an sich gültig sind, deren Energieverteilungskurve aber außerhalb der Grenzen des eingestellten Energiefensters liegt, durch die Energiekorrektur in das fest eingestellte Fenster geschoben werden.

Im Falle der Figur 3 wird also beispielsweise der Energieverteilungskurve 84 aus dem Flächenausschnitt 2 mit dem Korrekturfaktor für diesen Flächenausschnitt 2 so belegt, daß die gesamte Kurve um den Betrag $\Delta Z$ nach rechts verschoben ist. Die Verteilungskurve befindet sich damm innerhalb der Grenzen W1L, W1U des Fensters des Energie-Analysators 48. Es kann jetzt mit Hilfe des Fensters untersucht werden, ob die aus dem Flächenausschnitt 2 stammenden Ereignisse für die Aufzeichnung gültig sind oder ob sie möglicherweise nicht gültig sind.

Die Korrektur kann auch durchgeführt werden in Verbindung mit einer einzelnen Strahlungsquelle, die selbst eine Mehrzahl unterschiedlicher Energieniveaus aufweist. Ebensogut ist die Korrktur aber auch möglich, wenn die Bildereignisse von einer Mehrzahl von Strahlungsquellen stammen, die untereinander unterschiedliche Energieniveaus der Strahlung haben. Die Art der Korrektur bleibt dieselbe; es braucht lediglich zur Auswertung mehrerer Energieniveaus eine entsprechend hohe Zahl von Energie-Analysatoren mit entsprechend unterschiedlichen Energiefenstern vorgesehen zu werden. In der Figur 1 ist diese Möglichkeit durch Hinzufügung von zwei weiteren Energie-Analysatoren 90 und 92 angedeutet.

Nach Figure 1 werden die Energiekorrekturfaktoren, die im Speicher 54 gespeichert sind, während einer off-line Test- Meß- und Analysierphase errechnet, wozu der in der Figur 1 auf der linken Seite dargestellte off-line Korrekturfaktorapparat 100 dient. Dieser Apparat 100 wird von den X- und Y-Positionskoordinatensignalen an den Ausgängen 26 und 28 und vom Z-Energiesignal am Ausgang 40 der Szintillationskamera gespeist. Sobald eine entsprechend große Zahl von Ereignissen erfaßt ist, die eine gewisse statische Genauigkeit ermöglicht, werden die Bildereignisdaten analysiert und dadurch die nötigen Korrekturfaktoren ermittelt. Die Korrekturfaktoren werden dann über den Ausgang 102 des Apparates 100 zum Speicher 54 zwecks Einspeicherung in Form eines Datenfeldes vom Korrekturfaktoren für die kleinen Flächenausschnitte der Kamerafläche ausgegeben.

Wenden wir uns jetzt einer detaillierteren Diskussion über die Art zu, mit der die Korekturfaktoren in der off-line Test, Meß- und Analysierphase errechnet werden. Der Apparat 100 umfaßt Datenspeicher in denen die ermittelten Bildereignisse und auch die Wert der Energien der Bildereignissignale gespeichert werden, so wie sie während der off-line Test-, Meß- und Analysierphase anfallen. Die Zahl aller Bildereignisse, die erfaßt und eingespeichert wird und die eine gewisse statistische Sicherheit gibt, kann bis zu 20 Millionen betragen.

All anfallenden Bildereignisdaten werden also in dem Datenspeicherndes Apparates 100 gespeichert, und zwar in einer solchen Koordinatenposition, die jener entspricht, in der das Ereignis auftritt. Um dies realisieren zu können, wird die Bildfläche der Kamera in eine Matrix unterteilt, die 64 horizontale und 64 vertikale Linien aufweist. Die gesamte Kamerabildfläche ist damit in insgesamt 4096 Bildflächenelemente oder Flächenausschnitte aufgeteilt. Die Bildfläche einer Szintillationskamera ist normalerweise kreisförmig. Dies hat zur Folge, daß bei der quadratischen Form der 64×64 Matrix einzelne Bildflächenelemente außerhalb der kreisförmigen Kamerablindfläche liegen und deshalb unbenutzt bleiben. Dies ist jedoch bei der relative großen Zahl der tatsächlich benutzten Bildflächenelemente völlig ohne Bedeutung.

Im Meßbetrieb, in dem die Flächen-Matrix der Bildfläche der Kamera einer Flutungsquelle für radioaktive Strahlung ausgesetzt wird, wird, nun im Apparat 100 die Gesamtzahl $N_T$ aller Bildereignisse, die in einem Bildflächenelement auftreten, gezählt. Das Zählergebnis wird dann in einem Speicher des Apparates 100 abgespeichert. Zur Speicherung eines Zählergebnisses können dabei z.B. für jedes Flächenelement insgesamt 16 bits zur Verfügung gestellt werden. Gleichzeitig, während also die anfallenden Bildereignisse gezählt werden, wird auch deren Energie für jedes Flächenelement in einem Z-Speicher des Apparates 100 aufsummiert. Am Ende der Test- und Meßphase sind also in Speichern des Apparates 100 für jedes einzelne Flächenelement insgesamt zwei Informationen abgespeichert, nämlich die Zahl der jeweils angefallenen Bildereignisse in diesem Flächenelement einerseits und die Gesamtenergie der angefallenen Ereignisse dieses Flächenelements andererseits. Für die Energiespeicherung können dabei für jedes Flächenelement z.B. 24 bits zur Verfügung gestellt werden. Aus diesen Daten können nun die Energiekorrekturfaktoren für jedes Bildflächenelement berechnet werden, die ein Maß für die Ungleichförmigkeit der Kameraantwort auf eine gleichmäßige Durchstrahlung aller Bildflächenelemente auf der Kamerabildfläche mit gleicher Energie sind.

Die Berechnung wird dabei wie folgt durchgeführt:

Der Korrekturfaktorapparat 100 berechnet von den gespeicherten Daten die Durchschnittsenergie pro Ereignis $Z_{MAP}$ für jedes Bildflächenelement dadurch, daß er jeweils die Inhalte $Z_{FLD}$ des Gesamtenergiespeichers durch die Inhalte $N_T$ des Gesamtzahlspeichers dividiert:

$$Z_{MAP} = \frac{Z_{FLD}}{N_T} \qquad (1)$$

Von der Durchschnittsenergie $Z_{MAP}$ pro Ereignis wird ein Energiekorrekturfaktor f(P) berechnet, der für jedes Bildflächenelement und als prozentualer Korrekturfaktor den Betrag repräsentiert, um welchen die mittler Energie $Z_{MAP}$ aller Ereignisse für jedes Bildflächenelement von dem $Z_{MAP}$ Wert eines Referenzflächenelementes abweicht. Das Referenzflächenelement ist nach einer besonderen Ausgestaltung das zentrale Bildflächenelement der Kamerabildfläche, und sein mittlere Energiewert ist un Übereinstimmung mit der Fotovervielfacher-röhren-Anordnung als $Z_{19} = Z_{Ref}$ bezeichnet. Der Korrekturfaktor f(P) wird in Form eines variablen Korrekturfaktors f(P)', der in seinem Zentralbereich den Wert Null aufweist, wie folgt berechnet:

$$f(P)' = \frac{(Z_{19} - Z_{MAP})1000}{Z_{MAP}} \qquad (2)$$

Der Faktor 1000 repräsentiert dabei einen Maßstabsfaktor in Übereinstimmung mit den Parametern des Energiekorrekturgerätes 10 und zur leichteren digitalen Speicherung der Faktoren f(P) als binäre Zahlen in einem geeigneten Bereich im on-line Energiekorrekturfaktor-Speicher 54. Falls also f(P) z.B. als binäres Wort von 8 bits, das eine Zahl von 0 bis 356 repräsentiert, gespeichert ist, so entspricht der Korrekturfaktor f(P)' in einer Prozentualzahl ausgedrückt-einer Änderung der Energie $Z_{MAP}$ im Bereich von ±12,8% bezuglich $Z_{Ref} = Z_{19}$ und die Variable f(P)' umfaßt einen Bereich von −128 bis +127 mit einem Wert Null im Zentrumbereich, wobei gilt f(P)=f(P)'+128.

Die auf diese Weise für jedes Bildflächenelement errechneten f(P)-Werte werden dann im Speicher 54 des on-line Energiekorrekturgerätes 10 als ein 64×64 adressierbares Datenfeld von digitalen Daten gespeichert, genauso wie sie aus den Daten der off-line Test-, Meß- und Anslysierphase errechnet wurden. Das 64×64 Datenfeld repräsentiert an jedem Speicherort den Faktor f(P) für das entsprechende Bildelement. In einer bevorzugten Ausgestaltung ist der Korrekturfaktor f(P) als digitale Zahl von 0 bis 255 entsprechend einem Bereich der Variablen f(P)' von −128 bis +127 um den mit Null bezeichneten Referenzwert gespeichert. So wie er gespeichert ist, entspricht der Referenzwert Null einem Wert von 128 in einem Bereich von 0 bis 255.

Kehrt man nun zu einer detaillierteren Diskussion des on-line Energiekorrekturgerätes 10 zurück und bezieht sich jetzt auf die Figur 5, so werden die $X_a$- und $Y_a$-Koordinatenpositionen eines jeden Bildereignisses dazu benutzt, um den Korrekturfaktor f(P) für jenes Bildelement entsprechend zu adressieren und auszulesen, das dem Bildereignis vom Energiekorrekturfaktor-Speicher 54 entspricht. Die Energiesignal-Korrektureinheit 58 des Energiekorrekturgerätes 10 antwortet auf den ausgelesenen Korrekturfaktor f(P), modifiziert das unkorrigierte Energiesignal $Z_{IN}$ bei 40 und erzeugt korrigiertes $Z_C$-Signal bei 60.

Bevor auf eine nähere Betrachtung der Funktionsweise des Gerätes 10 anhand der Figur 5 eingegangen wird, werden zuerst einmal die Schaltkreiselemente und ihre internen Beziehungen in der Energiesignal-Korrektureinheit 58 diskutiert, um so die Arbeitsweise der Korrektureinheit 58 in Sicht auf die bereichneten und gespeicherten Werte f(P) besser verstehen zu können.

Der Energiesignal-Korrekturkreis 58 beinhaltet in einer besonderen Ausführung einen Operationsverstärker 110, der einen invertierenden Eingang, hat, der über einen ohmschen Widerstand 112 mit dem umkorrigierten $Z_{IN}$-Energiesignal 40 beaufschlagt ist. Ein Rückkoppelwiderstand 114 ist zwischen dem Ausgang und den invertierenden Eingang des Operationsverstärkers 110 geschaltet. Der nicht invertierende Eingang des Verstärkers 110 liegt an Erdbezugspotential 116. Die Widerstandswerte der ohmschen Widerstände 112 und 114 sind gleich groß unt mit R angegeben. Demnach ist der Verstärker 110 also ein invertierender Verstärker mit der Verstärkung Eins. Der Ausgang des Verstärkers 110 ist über einen ohmschen Widerstand 118 mit dem Wert 1,5 R am Analogbezugseingang eines multiplizierenden Digital-Analog-Konverters 120 (im nachfolgenden MDAC genannt) angeschaltet.

Der Digitaleingang des MDAC 120 wird mit den Korrekturfaktoren f(P) am Ausgang 56 des Energiekorrektur-Speichers 54 beaufschlagt. Der analoge Ausgang des MDAC 120 ist mit dem invertierenden Eingang eines Operationsverstärkers 122 verbunden. Ein Rückkoppelwiderstand 124 mit dem Wert $R_A$ ist zwischen Ausgang und invertierenden Eingang des Verstärkers 122 geschaltet. Der nicht invertierende Eingang des Verstärkers 122 liegt an Erdbezugspotential 116. Ein ohmscher Widerstand 126 mit dem Widerstandswert 3R ist zwischen den invertierenden Eingang des Verstärkers 122 und den Ausgang des Verstärkers 110 geschaltet. Der Ausgang des Verstärkers 110 ist außerdem über einen ohmschen Widerstand 128 mit dem Widerstandswert R zum invertierenden Eingang einer Operationsverstärker-Ausgangsstufe 130 gekoppelt. Am nicht invertierenden Eingang des Verstärkers 130 liegt Erdbezugspotential 116. Der invertierende Eingang des Verstärkers 130 ist über einen ohmschen Widerstand 131, der den Wert 2,615 R hat, an Ausgang des Verstärkers 122, dessen Ausgangssignal mit $V_c$ bezeichnet ist, angeschlossen. Ein Rückkoppelwiderstand 132 mit dem Widerstandswert R verbindet den Ausgang des Verstärkers 130 mit seinem invertierenden Eingang. Der Ausgang 60 des Verstärkers 130 liefert das korrigierte Energiesignal $Z_C$, das dem Energie-Analysator 48 der Figur 1 zugeleitet wird. Der Ausgangsstrom des MDAC 120 hat den Wert $I_o$.

Mit diesen Parametern kann dann eine Beziehung zwischen dem variablen Korrekturfaktor f(P)', dem korrigierten Energiesignal $Z_C$ bei 60 und dem unkorrigierten Energiesignal $Z_{IN}$ bei 40 in der Weise hergestellt werden, wie es weiter unten anhand der Gleichungen 4 bis 11 dargestellt ist. Man erhält damit die gewünschte Beziehung, die gewährleistet, daß die bei 40 eingehenden Bildereignis-Energiesignale so korrigiert werden, daß die Ungleichförmigkeiten der Energieantwort der Kamera anhand der bei 60 anfallenden korrigierter Energiesignale $Z_C$ beseitigt werden.

Für die Berechnung und Analyse, wie sie vorstehend diskutiert wurden, sind in bevorzugter Ausbildung die Korrekturfaktoren f(P) in einem Bereich von 0 bis 255 gespeichert, und sie sind in den folgenden Beziehungen als Variable f(P)' innerhalb des Bereiches −128 bis +127 vorausgesetzt. Damit ergibt sich ein zentraler unkorrigierter Nullwert für f(P)', der einem Wert für f(P) von 128 im Zentrum des Bereiches 0 bis 256, so wie er abgespeichert ist, entspricht:

$$-128 \leq f(P)' \leq 127 \qquad (4)$$

$$I_o = -\frac{f(P)'+128}{256} \cdot \frac{Z_{IN}}{1,5R} \qquad (5)$$

$$V_c = -R_A\left(-\frac{f(P)'+128}{256} \cdot \frac{Z_{IN}}{1,5R} + \frac{Z_{IN}}{3R}\right) \qquad (6)$$

$$V_c = R_A\frac{f(P)'Z_{IN}}{(256)(1,5R)} \qquad (7)$$

$$-Z_{OUT} = -Z_C = Z_{IN} + \frac{V_c}{2,615} = Z_{IN} + \frac{f(P)'Z_{IN}R_A}{(256)(1,5R)(2,615)} \qquad (8)$$

$$-Z_{OUT} = -Z_C = Z_{IN}\frac{1+f(P)'R_A}{(256)(1,5R)(2,615)} \qquad (9)$$

Um nun f(P)' so ermitteln zu können, daß $Z_{OUT} = Z_C = Z_{IN}$ für $Z_{Ref}$ beim Referenzsignal des Fotorvervielfachers 19 ist, muß $Z_{19} = 0$ (entsprechend einem Nullkorrekturfaktor) wie folgt gewählt sein:

$$Z_{19} = Z_{IN} + \frac{Z_{IN}f(P)'R_A}{1004R} \qquad (10)$$

Wird nach f(P)' aufgelöst, so ergibt sich:

$$f(P)' = \frac{Z_{19}-Z_{IN}}{Z_{IN}} \cdot \frac{1004}{(R_A/R)} \qquad (11)$$

Falls also f(P)' gemäß der Beziehung der Gleichung (2) errechnet wird, wird $R_A/R$ in der Energiesignal-Korrektureinheit 58 so ausgewählt oder einjustiert, daß $R_A/R=1{,}004$.

Wenn also die (gespeicherten Korrekturfaktor en f(P) einen Bereich von 0 bis 255 haben und der zentral Nullwert des Bereiches bei 128 liegt, ergibt sich die Beziehung:

$$f(P)=f(P)'+128, \qquad (12)$$

wobei

$$f(P)=\frac{Z_{19}-Z_{IN}}{Z_{IN}} \cdot \frac{1004}{R_A/R}+128, \qquad (13)$$

und mit $R_A/R=1{,}004$:

$$f(P)=\frac{Z_{19}-Z_{IN}}{Z_{IN}} \cdot 1000+128. \qquad (14)$$

So ergibt sich also, daß die Beziehungen (2) und (14) in Übereinstimmung gebracht sind bei der Berechnung und Abspeicherung des Korrekturfaktors f(P) und beim Gebrauch des Korrekturfaktors f(P) durch die Energiesignal-Korrektureinheit 58 zum Zwecke der Korrektur des Bildereignis-Energiesignals $Z_{IN}$. Wenn ein Bildereignis anfällt, wird das nicht korrigierte Signal $Z_{IN}$ der Energiesignal-Korrektureinheit 58 zugeführt. Gleichzeitig wird in Einklang mit der X-Y-Koordinatenposition des Bildereignisses, von dem das $Z_{IN}$-Signal stammt, der f(P)-Korrekturfaktor vom Speicher 54 zur Einheit 58 ausgelesen. Es wird somit am Ausgang des MDAC 120 ein Korrekturfaktorsignal erhalten, das maßstäblich entsprechend dem Wert des Bildereignis-Energiesignals $Z_{IN}$ eingestellt ist. Das Ausgangssignal des MDAC 120 wird dann vom Verstärker 122 zum Ausgangssignal $V_C$ weiterverarbeitet. Das $V_C$-Signal wird dann dem $Z_{IN}$-Signal am invierenden Eingang des Verstärkers 130 hinzugezählt. Das $V_C$-Signal wird anschließend vom Widerstand 131 mit dem Wert 2,615 R skaliert. Damit wird also der Korrekturfaktor f(P) durch den aktuellen Energiewert des Bildereignisses gewichtet und der so erhaltene Wert zum Bildereignis-Energiesignal $Z_{IN}$ hinzugefügt, so daß ausgangsseitig ein korrigiertes Energiesignal $Z_C$ in Proportionalität zum Eingangssignal und geändert mit dem Korrekturfaktor f(P) erhalten wird.

Auf diese Weise korrigiert also das on-line Energiekorrekturgerät 10 das eingangsseitige Bildereignis-Energiesignal $Z_{IN}$ von Ereignis zu Ereignis entsprechend der ungleichförmigen Energieantwort der Kamera. Das Ergebnis ist das korrigierte Energiesignal $Z_C$, das vom Energie-Analysator 48 analysiert wird dahingehend, ob das Ereignis zur Anzeige angenommen oder ob es zurückgewiesen wird.

Bei diesem Sachverhalt ergibt sich also, daß ein Einsatz des on-line Energiekorrekturgerätes 10 möglich ist sowohl im Zusammenhang mit einer einzigen Strahlungsquelle, die eine Mehrzahl von strahlenden Energieniveaus hat, als auch im Zusammenhang mit mehreren Strahlungsquellen, die voneinander unterschiedliche Energieniveaus haben. Es brauchen den Bildereignis-Energiesignalen, die in den unterschiedlichen Bereichen der Energieniveaus erzeugt werden lediglich die entsprechend vorher ermittelten Korrekturfaktoren hinzugefügt zu werden. Die Entscheidung darüber, ob ein Ereignis entsprechend den unterschiedlichen Energie niveaus als gültig oder als ungültig zu zählen ist, wird dann von einer entsprechenden Zahl von Energie-Analysatoren getroffen, deren Energiefenster auf die erwünschten vielfältig unterschiedlichen Energieniveaus der Quelle oder der Quellen eingestellt sind. Ein Eingriff in den Aufbau der Szintillationskamera ist also nicht notwendig.

Vorstehende Beschreibung betrifft lediglich ein Aufführungsbeispiel der Erfindung. Es ist selbstverständlich, daß es durchaus Modifikationen dieses Ausführungsbeispiels gibt, die alle mit in den Bereich der Erfindung fallen. Im vorliegenden Ausführungsbeispiel liefert die Energie-Korrektureinheit 58 z.B. eine exakt linearisierte Antwort. Es ist aber selbstverständlich, daß auch andersartige Korrektureinheiten, z.B. auf der Basis digitaler und analoger Rechner und Funktionsgeneratoren, eingesetzt werden können, die eine Energiekorrektur des Bildereignis-Energiesignals $Z_{IN}$ durchführen können, welche Korrektur der grundsätzlichen Beziehung $Z_C=Z_{IN} \cdot (1+f(P)')$ folgt, vergl. Beziehung (9). So kann z.B. ein Digitalrechner dazu eingesetzt werden, eine Modifikation des Energiesignals entsprechend dem gespeicherten Korrekturfaktor durchzuführen, wobei eine die Interpolation des gespeicherten korrigierten Faktors in Abhängigkeit von der Position des Bildereignisses eingeschlossen ist. Auch ist es selbstverständlich, daß in besonderen Ausführungsformen die Korrekturfaktoren f(P) in den verschiedenartigsten Formaten und Bereichen gespeichert sein können und daß sie mit Einrichtungen geändert werden können, die nicht multiplizierende Digital-Analog-Konverter sind.

**Patentansprüche**

1. Verfahren zur Korrektur von Bildereignis-Energiesignalen einer Szintillationskamera in bezug auf Ungleichförmigkeiten in der Energieverteilungs-Charakteristik der Szintillationskamera, welche Ungleichförmigkeiten eine Funktion der Bildfeldposition der Bildereignisse sind, sowie zur Überprüfung der korrigierten Bildereignis-Energiesignale,

a) wobei die Szintillationskamera neben dem Bildereignis-Energiesignal auch ein Bildereignis-Positionskoordinatensignal in Antwort auf jedes auftretende Bildereignis erzeugt,

b) wobei wenigstens ein vorgebene Grenzen aufweisendes Energiefenster erzeugt wird, das außerhalb der vorgegebenen Grenzen liegende Bildereignis-Energiesignale verwirft,

c) wobei eine Matrix von Korrekturfaktoren während einer off-line Test-, Meß- und Analysierphase bestimmt wird, indem die energieabhängige Antwort der Szintillationskamera auf eine eine gleichmäßige Strahlungsverteilung aufweisende Teststrahlungsquelle gemessen wird, wobei die Korrekturfaktoren jeweils eine Änderung der energieabhängigen Antwort der Szintillationskamera als Funktion des Bildfeldposition repräsentieren,

d) wobei die so bestimmten Korrekturfaktoren zum Gebrauch während einer on-line Untersuchungsphase der Szintillationskamera gespeichert werden,

e) wobei während der on-line Untersuchungsphase jeweils derjenige Korrekturfaktor, der zum gerade gebildeten Bildereignis-Positionskoordinatensignal gehört, ausgelesen wird, dadurch gekennzeichnet,

f) daß jeweils das Bildereignis-Energiesignal für das betreffende Bildereignis dadurch korrigiert wird daß das Bildereignis-Energiesignal ($Z_{IN}$) mit dem gerade ausgelesenen Korrekturfaktor ($f(P)'$) multipliziert und daß zu dem Produkt ($f(P)' \cdot Z_{IN}$) das Bildereignis-Energiesignal ($Z_{IN}$) addiert wird, wodurch ein korrigiertes Bildereignis-Energiesignal ($Z_C$) erhalten wird, und

g) daß dieses korrigierte Bildereignis-Energiesignal ($Z_C$) dem Energiefenster zugeleitet wird, welches fest eingestellte Grenzen aufweist.

2. Vorrichtung zur Durchführung des Verfahrens nach Anspruch 1, mit einer Szintillationskamera, die Bildereignis-Positionskoordinatensignale ($X$, $Y$) und Bildereignis-Energiesignale ($Z_{IN}$) für diejenigen Bildereignisse liefert, die während der on-line Untersuchungsphase der Szintillationskamera auftreten, mit einem Speicher (54), in dem in der off-line Test-, Meß- und Analysierphase ermittelte Korrekturfaktoren (f(P)) in einer adressierbaren Daten-Matrix gespeichert sind, wobei diese Korrekturfaktoren (f(P)) jeweils eine Änderung der energieabhängigen Antwort der Szintillationskamera als Funktion der Bildfeldposition repräsentieren, mit einer Steuereinheit (50, 52), die auf das Bildereignis-Positions-koordinatensignal ($X$, $Y$) eines jeden Bildereignisses hin den Speicher (54) in der Weise steuert, daß er den Korrekturfaktor (f(P)) entsprechend dem gerade anliegenden Bildereignis-Positionskoordinatensignal ($X$, $Y$) ausliest, mit einer Korrektureinheit (58) für das Bildereignis-Energiesignal ($Z_{IN}$), die in Abhängigkeit vom Bildereignis-Energiesignal ($Z_{IN}$) und dem ausgelesenen Korrekturfaktor (f(P)) ein korrigiertes Bildereignis-Energiesignal ($Z_C$) liefert, und mit einem Energie-Analysator (48), der ein Energiefenster mit vorgegebenen Grenzen (W1L, W1U) aufweist, dem das korrigierte Bildereignis-Energiesignal ($Z_C$) zugeführt ist und der außerhalb der vorgegebenen Grenzen (W1L, W1U) liegenden Bildereignis-Energiesignale ($Z_{IN}$) verwirft, dadurch gekennzeichnet, daß die Korrektureinheit (58) Multipliziermittel (120) umfaßt, um den ausgelesenen Korrekturfaktor (f(P)) mit dem Bildereignis-Energiesignal ($Z_{IN}$) zu multiplizieren, sowie Mittel (130) zur Addition, um das Bildereignis-Energiesignal ($Z_{IN}$) und das Produkt ($V_C$) aus dem Bildereignis-Energiesignal ($Z_{IN}$) und dem ausgelesenen Korrekturfaktor (f(P)) zum korrigierten Bildereignis-Energiesignal ($Z_C$) zusammenzufassen, und daß der Energie-Analysator (48) ein Energiefenster mit fest eingestellten Grenzen (W1L, W1U) aufweist.

3. Vorrichtung nach Anspruch 2, dadurch gekennzeichnet, daß die ermittelten Korrekturfaktoren (f(P)) prozentuale Korrekturfaktoren sind, die für jede Bildfeldposition den Betrag repräsentieren, um welchen die mittlere Energie ($Z_{MAP}$ eines bildereignisses von der mittleren Energie eines Bildereignisses in einer Referenz-Bildfeldposition abweicht.

4. Vorrichtung nach Anspruch 2 oder 3, dadurch gekenzeichnet, daß die Korrekturfaktoren (f(P)) in digitaler Form vorliegen und die Multipliziermittel einen multiplizierenden Digital-Analog-Konverter (120) umfassen, dem die Korrekturfaktoren (f(P)) am digitalen Eingang und das Bildereignis-Energiesignal ($Z_{IN}$) an einem multiplizierenden Analog-Referenzeingang zugeleitet sind, und daß die Mittel (130) zur Addition eingangsseitig mit dem Bildereignis-Energiesignal ($Z_{IN}$) und mit dem Ausgangssignal ($V_C$) des multiplizierenden Digital-Analog-Konverters (120) beaufschlagt sind.

5. Vorrichtung nach einem der Ansprüche 2 bis 4, dadurch gekennzeichnet, daß zur Bestimmung der Korrekturfaktoren (f(P)) während der off-line Test-, Meß- und Analysierphase ein Korrekturfaktorapparat (100) vorgesehen ist.

6. Verfahren nach Anspruch 1, dadurch gekennzeichnet, daß die Energieverteilungs-Charakteristik der Szintillationskamera unter Einsatz einer Flutungsquelle gemessen wird und daß ein Korrekturfaktorapparat (100) in der off-line Test-, Meß- und Analysierphase daraus die Korrekturfaktoren (f(P)) errechnet.

7. Verfahren nach Anspruch 6, dadurch gekennzeichnet, daß die Korrekturfaktoren (f(P)) in der Weise errechnet werden, daß die Gesamtzahl ($N_T$) aller Bildereignisse in jeder Bildfeldposition gezählt und die Gesamtenergie ($Z_{FLD}$) aller Bildereignisse in der jeweiligen Bildposition aufsummiert wird und daß anschließend für jede Bildfeldposition die Gesamtenergie ($Z_{FLD}$) durch die Gesamtzahl ($N_T$) aller Bildereignisse dividiert wird, wodurch jeweils ein mittlerer Energiewert ($Z_{MAP}$) erhalten wird, und daß der Korrekturfaktor (f(P)) für eine jede Bildfeldposition als Verhältnis zwischen dem jeweiligen mittleren Energiewert ($Z_{MAP}$) und dem mittleren Energiewert für eine Referenz-Bildfeldposition errechnet wird.

8. Vorrichtung nach einem der Ansprüche 2 bis 5, dadurch gekennzeichnet, daß die Szintillationskamera eine Mehrzahl von Energie-Analysatoren (48, 90, 92) umfaßt, von denen jeder ein Energiefenster mit festgelegten Grenzen hat, um unterschiedliche Energieniveaus des korrigierten Bildereignis-Energiesignals ($Z_C$) messen zu

können, wobei das korrigierte Bilderreignis-Energiesignal ($Z_C$) dieser Mehrzahl von Energie-Analysatoren (48, 90, 92) zugeleitet ist, und wobei die Korrektureinheit (58) für das korrigierte Bildereignis-Energiesignal ($Z_C$) in der Weise arbeitet, daß sie die Ungleichförmigkeiten der Szintillationskamera für Bildereignisse, die von einer einzigen Energiequelle mit einer Vielzahl von Energieniveaus oder die von einer Mehrzahl von Energiequellen mit unterschiedlichen Energieniveaus herrühren, korrigiert.

9. Vorrichtung nach einem der Ansprüche 2 bis 5 oder 8, dadurch gekennzeichnet, daß die Korrekturfaktoren (f(P)) im Speicher (54) digital gespeichert sind, wobei der Datenbereich einen Bereich von 0 bis $2^N$ einnimmt, dessen Zentrum einem Korrekturfaktor (f(P)′) Null entspricht.

## Revendications

1. Procédé pour corriger la réponse en énergie à un évènement d'image d'une caméra à scintillation en rapport avec des irrégularités de la caractéristique de distribution d'énergie de la caméra à scintillation, lesquelles irrégularités sont fonction de la position des évènements d'image dans le champ d'image, ainsi que pour contrôler la réponse en énergie corrigée à un évènement d'image,

a) selon lequel la caméra à scintillation produit, endehors de la réponse en énergie à un évènement d'image, également un signal de coordonnées de position d'évènement d'image en réponse à chaque évènement d'image apparaissant,

b) et selon lequel au moins une fenêtre d'énergie, qui possède des limites prédéterminées et qui rejette la réponse en énergie à un évènement d'image se situant à l'extérieur des limites prédéterminées,

c) et selon lequel une matrice de facteurs de correction est déterminée pendant une phase d'essai, de mesure et d'analyse en ligne, par le fait qu'on mesure la réponse, dépendant de l'énergie, de la caméra à scintillation à une source de rayonnement de test possédant une distribution de rayonnement uniforme, les facteurs de correction représentant respectivement une variation de la réponse, dépendant de l'énergie, de la caméra à scintillation en fonction de la position dans le champ d'image,

d) et selon lequel les facteurs de correction ainsi déterminés sont mémorisés en vue d'être utilisés pendant une phase d'examen en ligne de la caméra à scintillation,

e) et selon lequel pendant la phase d'examen en ligne, le facteur de correction, qui est associé au signal de coordonnées de position d'évènement d'image précisément formé, est extrait par lecture,
caractérisé par le fait que:

f) respectivement la réponse en énergie à un évènement d'image pour l'évènement d'image concerné est corrigée par le fait que la réponse en énergie ($Z_{IN}$) d'un évènement d'image est multipliée par le facteur de correction (f(P)′) extrait précisément par lecture, et qu'au produit (f(P)′ · $Z_{IN}$) est ajoutée la réponse en énergie ($Z_{IN}$) à un évènement d'image, ce qui permet d'obtenir une réponse en énergie corrigée ($Z_C$) à un évènement d'image, et

g) que cette réponse en énergie corrigée ($Z_C$) à une évènement d'image est envoyée à la fenêtre d'énergie, qui possède des limites réglées de façon fixe.

2. Dispositif pour la mise en oeuvre du procédé suivant la revendication 1, comportant une caméra à scintillation, qui délivre des signaux (X, Y) de coordonnées de position d'un évènement d'image et des réponses en énergie ($Z_{IN}$) à une évènement d'image pour les évènement d'image qui apparaissent pendant la phase d'examen en ligne de la caméra de scintillation, une mémoire (54) dans laquelle des facteurs de correction (f(P)) déterminés lors de la phase d'essai, de mesure et d'analyse en ligne, sont mémorisés dans une matrice de données adressables, ces facteurs de correction (f(P)) représentant respectivement une variation de la réponse, dépendant de l'énergie, de la caméra à scintillation en fonction de la position dans le champ d'image, une unité de commande (50, 52) qui, lors de l'apparition d'un signal (X, Y) de coordonnées de position de chaque évènement d'image, commande la mémoire (54) de manière qu'elle extrait par lecture le facteur de correction (f(P)) conformément au signal (X, Y) de coordonnées de position d'un évènement d'image, précisément appliqué, une unité de correction (58) pour la réponse en énergie ($Z_{IN}$) à un évènement d'image et qui fournit une réponse en énergie corrigée ($Z_C$) à un évènement d'image, en fonction de la réponse en énergie ($Z_{IN}$) à une évènement d'image et du facteur de correction (f(P)) extrait par lecture, et un analyseur d'énergie (48) qui possède une fenêtre d'énergie possédant des limites prédéterminées (W1L, W1U) et auquel la réponse en énergie corrigée ($Z_C$) à un évènement d'image est envoyée et qui rejette la réponse en énergie ($Z_{IN}$) à un évènement d'image, qui est située à l'extérieur des limites prédéterminées (W1L, W1U), caractérisé par le fait que l'unité de correction (58) contient des moyens multiplicateurs (120) permettant de multiplier le facteur de correction (f(P)) extrait par lecture, à la réponse en énergie ($Z_{IN}$) à un évènement d'image, ainsi que des moyens (130) servant à réaliser une addition, pour réunir la réponse en énergie ($Z_{IN}$) à un évènement d'image et le produit ($V_C$) formé à partir de la réponse en énergie ($Z_{IN}$) à un évènement d'image et du facteur de correction (f(P)) extrait par lecture, pour former la réponse en énergie corrigée ($Z_C$) à un évènement d'image, et que l'analyseur d'énergie (48) comporte une fenêtre d'énergie possédant des limites (W1L, W1U) réglées de façon fixe.

3. Dispositif suivant la revendication 2, caractérisé par le fait que les facteurs de correction (f(P)) calculés sont des facteurs de correction en pourcentage, qui représentent pour chaque position du champ d'image la valeur dont l'énergie moyenne ($Z_{MAP}$) d'un évènement d'image s'écarte de l'énergie moyenne d'un évènement d'image dans sa position de référence dans le champ d'image.

4. Dispositif suivant la revendication 2 ou 3, caractérisé par le fait que les facteurs de correction (f(P)) sont présents sous forme numérique et que les moyens multiplicateurs incluent un convertisseur numérique/analogique (12) réalisant une multiplication, à l'entrée numérique duquel les facteurs de correction (f(P)) sont envoyés et à une entrée de référence analogique multiplicatrice duquel est envoyée la réponse en énergie ($Z_{IN}$) à un évènement d'image, et que les moyens additionneurs (130) sont chargés, du côté entrée, par la réponse en énergie ($Z_{IN}$) à l'évènement d'image et par le signal de sortie ($V_C$) du convertisseur numérique/analogique (120) effectuant une multiplication.

5. Dispositif suivant l'une des revendications 2 à 4, caractérisé par le fait que pour la détermination des facteurs de correction (f(P)) pendant la phase d'essai, de mesure et d'analyse en ligne, il est prévu un appareil (100) délivrant les facteurs de correction.

6. Procédé suivant la revendication 1, caractérisé par le fait qu'on mesure la caractéristique de distribution de l'énergie de la caméra de scintillation moyennant l'utilisation d'une source de flux et qu'un appareil (100) délivrant les facteurs de correction calcule, à partie de là, le facteur de correction (f(P)), lors de la phase d'essai, de mesure et d'analyse en ligne.

7. Procédé suivant la revendication 6, caractérisé par le fait que les facteurs de correction (f(P)) sont calculés de telle sorte que le nombre total ($N_T$) de tous les évènements d'image dans chaque position à l'intérieur du champ d'image est calculé et que l'énergie totale ($Z_{FLD}$) de tous les évènement d'image dans la position d'image respective est formée par addition et qu'ensuite, pour chaque position dans le champ d'image, l'énergie total ($Z_{FLD}$) est divisée par le nombre total ($N_T$) de tous les évènements d'image, ce qui permet d'obtenir effectivement une valeur d'énergie moyenne ($Z_{MAP}$), et que le facteur de correction (f(P)) est calculé pour chaque position dans le champ d'image en tant que rapport entre la valeur d'énergie moyenne respective ($Z_{MAP}$) et la valeur moyenne d'énergie pour une position de référence du champ d'image.

8. Dispositif suivant l'une des revendication 2 à 5, caractérisé par le fait que la caméra à scintillation comporte une multiplicité d'analyseurs d'énergie (48, 90, 92), dont chacun possède une fenêtre d'énergie comportant des limites fixées, afin de permettre la mesure de différents niveaux d'énergie de la réponse en énergie corrigée ($Z_C$) à un évènement d'image, auquel cas la réponse en énergie corrigée ($Z_C$) à un évènement d'image est envoyée à cette multiplicité d'analyseurs d'énergie (48, 90, 92), et l'unité de correction (58) pour la réponse en énergie corrigée ($Z_C$) à un évènement d'image fonctionne de telle sorte qu'elle corrige les irrégularités de la caméra à scintillation pour des phénomènes d'image, qui proviennent d'une seule source d'énergie possédant un grand nombre de niveaux d'énergie ou bien qui proviennent d'une multiplicité de sources d'énergie possédant des niveaux d'énergie différents.

9. Dispositif suivant l'une des revendications 2 à 5 ou 8, caractérisé par le fait que les facteurs de correction (f(P)) sont mémorisés sous forme numérique dans la mémoire (54), la zone des données occupant une étendue allant de 0 à $2^N$, dont le centre correspond à un facteur de correction (f(P)') nul.

**Claims**

1. A method of correcting image event energy signals in a scintillation camera with regard to non-uniformities in the energy-distribution characteristic of the scintillation camera, which non-uniformities are a function of the image field position of the image events, and for checking the corrected image event energy signals,

a) wherein the scintillation camera also produces an image event position coordinate signal in response to each occurring image event in addition to the image event energy signal,

b) wherein at least one energy window is produced which has predetermined boundaries and which rejects image event energy signals which lie outside the predetermined boundaries,

c) wherein a matrix of correction factors is determined during an off-line testing, measuring and analysing phase, in that the energy-dependent response of the scintillation camera to a test radiation source which has an uneven radiation distribution is measured, where the correction factors respectively represent a change of the energy-dependent response of the scintillation camera as a function of the image field position,

d) wherein the correction factors determined in this manner are stored for use during an on-line testing phase of the scintillation camera,

e) wherein during the on-line testing phase that correction factor assigned to the currently formed image event position coordinate signal is read out,

characterised in

f) that the image event energy signal is corrected for the respective image event, the image event energy signal ($Z_{IN}$) being multiplied by the currently read-out correction factor (f(P)'), and the image event energy signal ($Z_{IN}$) added to the product ($f(P)' \cdot Z_{IN}$), whereby a corrected image event energy signal ($Z_C$) is obtained, and

g) that the corrected image event energy signal ($Z_C$) is supplied to the energy window which has firmly adjusted boundaries.

2. A device for implementing the method as claimed in Claim 1 comprising a scintillation camera which supplies image event position coordinate signals (X, Y) and image event energy signals ($Z_{IN}$) for those image events which occur during the on-line testing phase of the scintillation camera, comprising a store (54) in which correction factors (f(P)) determined in the off-line testing measuring and analysing phase are stored in an addressable data matrix, where the correction factors (f(P)) respectively represent a change in the energy-dependent response of the scintillation camera as function of the image field position, comprising a control unit (50, 52) which controls the store (54) as a result of the image event position coordinate signal (X, Y) of each image event in such a manner that it reads out the correction factor (f(P)) in accordance with the currently present image event position coordinate signal (X, Y), comprising a correction unit (58) for the image event energy signal $(Z_{IN})$, which supplies a corrected image event energy signal $(Z_C)$, in dependence upon the image event energy signal $(Z_{IN})$ and the read-out correction factor (f(P)), and comprising an energy analyser (48) which has an energy window with predetermined boundaries (W1L, W1U), to which the corrected image event energy signal $(Z_C)$ is supplied and which rejects image event energy signals $(Z_{IN})$ which lie outside the predetermined boundaries (W1L, W1U), characterised in that the correction unit (58) comprises multiplying means (120) in order to multiply the read-out correction factor (f(P)) by the image event energy signal $(Z_{IN})$, and means (130) for addition in order to combine the image event energy signal $(Z_{IN})$ and the product $(V_C)$ consisting of the image event energy signal $(Z_{IN})$ and the read-out correction factor (f(P)) to form the corrected image event energy signal $(Z_C)$, and that the energy analyser (48) has an energy window with firmly adjusted boundaries (W1L, W1U).

3. A device as claimed in Claim 2, characterised in that the determined correction factors (f(P)) are percentage correction factors which represent the amount for each image field position, by which the average energy $(Z_{MAP})$ of an image event differs from the average energy of an image event in a reference image field position.

4. A device as claimed in Claim 2 or 3, characterised in that the correction factors (f(P)) are present in the digital form and the multiplying means comprise a multiplying digital-analogue converter (120), to which the correction factors (f(P)) are supplied at the digital input and the image event energy signal $(Z_{IN})$ is supplied at a multiplying analogue reference input, and that the means (130) for addition are acted upon by the image event energy signal $(Z_{IN})$ and by the output signal $(V_c)$ of the multiplying digital-analogue converter (120).

5. A device as claimed in one of Claims 2 to 4, characterised in that a correction factor apparatus (100) is provided in order to determine the correction factors (f(P)) during the off-line testing, measuring and analysing phase.

6. A method as claimed in Claim 1, characterised in that the energy distribution characteristic of the scintillation camera is measured by using a flood source and that a correction factor apparatus (100) calculates the correction factors (f(P)) therefrom during the off-line testing, measuring and analysing phase.

7. A method as claimed in Claim 6, characterised in that the correction factors (f(P)) are calculated in such a manner that the total number $(N_T)$ of all image events is counted in each image field position and the total energy $(Z_{FLD})$ of all image events in the respective image position is summed up, and that for each image field position the total energy $(Z_{FLD})$ is subsequently divided by the total number $(N_T)$ of all image events, whereby an average energy value $(Z_{MAP})$ is respectively obtained, and that the correction factor (f(P)) for each image field position is calculated as the ratio between the respective average energy value $(Z_{MAP})$ and the average energy value for a reference image field position.

8. A device as claimed in one of Claims 2 to 5, characterised in that the scintillation camera comprises a plurality of energy analysers (48, 90, 92), each of which has an energy window with fixed boundaries in order to be able to measure different energy levels of the corrected image event energy signal $(Z_C)$, where the corrected image event energy signal $(Z_C)$ is fed to the plurality of energy analysers (48, 90, 92), and where the correction unit (58) for the corrected image event energy signal $(Z_C)$ operates in such a manner that it corrects the non-uniformities of the scintillation camera for image events, which emanate from one single energy source having a plurality of energy levels or from a plurality of energy sources having different energy levels.

9. A device as claimed in one of Claims 2 to 5 or 8, characterised in that the correction factors (f(P)) are digitally stored in the store (54), where the data range covers a range of 0 to $2^N$, whose centre corresponds to a correction factor (f(P)') of zero.

FIG 1

FIG 2

FIG 3

FIG 4

FIG 5